# FASCICULE DE BREVET EUROPEEN

(11) **EP 1 140 963 B1**
(45) Date de publication et mention de la délivrance du brevet: **22.09.2004**
(21) Numéro de dépôt: 00900527.3
(22) Date de dépôt: 05.01.2000
(51) Int. Cl.: C07H 19/10, C07H 19/20, C07H 21/00, C12Q 1/68, C12P 19/34

(54) **COMPOSE FONCTIONNALISE, POLYNUCLEOTIDE EVENTUELLEMENT MARQUE ET PROCEDE DE DETECTION D'UN ACIDE NUCLEIQUE CIBLE**
FUNKTIONALISIERTE VERBINDUNG, GEGEBENENFALLS MARKIERTE POLYNUKLEOTIDE UND VERFAHREN ZUR DETEKTION EINER ZIEL-NUKLEINSÄURE
FUNCTIONALISED POLYNUCLEOTIDE COMPOUND, OPTIONALLY MARKED AND METHOD FOR DETECTING A TARGET NUCLEIC ACID

(30) Priorité: 05.01.1999 FR 9900111
(43) Date de publication de la demande: 10.10.2001
(73) Titulaire: BIO MERIEUX, 69280 Marcy l'Etoile (FR)
(72) Inventeur: DEFRANC, Eric, F-38570 Concelin (FR); LAAYOUN, Ali, F-69007 Lyon (FR); LHOMME, Jean, F-38240 Meylan (FR); TREVISIOL, Emmanuelle, F-38000 Grenoble (FR)
(74) Mandataire: Guerre, Dominique
(86) Numéro de dépôt international: PCT/FR2000/000011
(87) Numéro de publication internationale: WO 2000/040590

(56) Documents cités:
- WO-A-92/00989
- WO-A-95/24185
- WO-A-95/32980
- WO-A-98/05766
- KISHORE N. S. ET AL: "Comparison of the acyl chain specificities of human myristoyl-CoA synthetase and human myristoyl-CoA: protein N-myristoyltransferase" THE JOURNAL OF BIOLOGICAL CHEMISTRY, vol. 268, no. 7, 1993, pages 4889-4902, XP002140156
- BADMAN, GEOFFREY T. ET AL: "Reactions between methiodides of nucleoside Mannich bases and carbon nucleophiles" J. CHEM. SOC., CHEM. COMMUN. (1987), (22), 1732-4, XP002117163

## Description

La présente invention concerne un nouveau composé nucléosidique ou nucléotidique, fonctionnalisé par un groupement alkylcétone, un polynucléotide comprenant au moins une unité nucléotidique fonctionnalisée par un groupement alkylcétone, avant et après marquage, ainsi que la mise en oeuvre et les applications de ces produits notamment pour la détection de séquences d'acide nucléique.

Dans le domaine des acides nucléiques, la synthèse de nucléotides fonctionnalisés a été décrite notamment dans le domaine du diagnostic et plus particulièrement pour la préparation de sondes nucléiques marquées utilisables pour la détection d'un acide nucléique cible.

Deux types de problèmes principaux se posent pour l'obtention de ces sondes. Dans un premier temps, le nucléotide fonctionnalisé doit être incorporé dans un polynucléotide. Ensuite, la fonction portée par le nucléotide doit pouvoir réagir sur un traceur de manière spécifique et efficace pour l'utilisation du polynucléotide comme sonde de détection.

Ainsi, le brevet EP-A-0 407 816 décrit des dérivés de l'uracile modifiés en position 5 pour la fabrication de sondes par voie chimique ou enzymatique. Toujours dans le même but, les brevets WO-A-86/06726 et EP-A-0 212 951 décrivent des dérivés de la cytosine modifiés en position 4. Le brevet EP-A-0 254 646 décrit un dérivé d'adénosine modifié en position 8.

Le brevet WO-A-92/00989 décrit une application particulière de nucléotides modifiés pour l'introduction de protéines sur un polynucléotide.

La demande de brevet WO-A-98/05766 de la demanderesse pose le problème de l'incorporation de nucléotides fonctionnalisés pouvant être incorporés par réaction enzymatique et notamment par les techniques d'amplification enzymatique, non plus pour la préparation de sondes marquées, mais directement pour générer une cible marquée. Dans ce cas, le besoin en sensibilité est plus important et le choix du nucléotide fonctionnalisé est crucial pour obtenir la sensibilité adéquate.

Un certain nombre de fonctions nucléophiles comme les fonctions amine et alcoxyamine, ou électrophiles comme la fonction aldéhyde sont décrites, qui permettent une incorporation efficace du nucléotide fonctionnalisé au cours de l'amplification, mais il subsiste néanmoins un besoin de nucléotide fonctionnalisé encore plus efficace, notamment en terme de facilité de préparation, en terme de neutralité vis à vis des réactions enzymatiques ou chimiques et en terme de réactivité pour le marquage de ce nucléotide par un traceur après incorporation.

Il a été trouvé par la demanderesse que la fonction alkylcétone répondait de manière surprenante aux inconvénients précités.

En effet, si l'art antérieur décrit l'utilisation de motifs R-CO- où R est un groupement alkyle dans le cas de la synthèse d'oligonucléotides (WO-A-93/22326), le but de cette fonction est de protéger l'amine exocyclique des bases. A la fin de la synthèse, l'amine est déprotégée par action d'un agent alcalin.

A titre d'illustration de cet état de la technique, on peut citer les documents suivants. L'article de K.K. Ogilvie et M.J. Nemer, Tetrahedron Letters, vol. 21, (1980) pages 4145-4148, divulgue, en tant qu'intermédiaire de synthèse d'un nucléotide, un composé nucléotidique porteur d'un groupement lévulinoyle fixé sur la fonction hydroxylée en 3' du pentose. L'introduction de ce groupement a pour but de protéger la fonction OH, et est ensuite aussitôt retirée après obtention dudit composé. L'article de C.T.J. Wreesmann et al., Nucleic Acids Research, vol. 11, N°23, (1983) pages 8389-8405, décrit l'obtention d'un intermédiaire dinucléotidique de synthèse dont les fonctions hydroxyle sont protégées par le groupement lévulinoyle. En présence d'ammoniaque, les fonctions hydroxyle sont libérées.

La fonction alkylcétone telle que définie dans la présente invention est suffisamment stable pour résister à ce type de traitement, ne représente donc pas un groupement protecteur et sa stabilité par rapport aux différentes méthodes de synthèse chimique a pour conséquence une facilité plus grande de préparation notamment par rapport à des fonctions amines, aldéhydes ou alcoxyamines comme décrit dans le brevet WO-A-98/05766.

De même dans les brevets cités précédemment (EP-A-0 407 816, WO-A-86/06726, EP-A-0 212 951, EP-A-0 254 646 et WO-A-92/00989) les fonctions décrites pour fonctionnaliser les nucléotides sont choisies parmi les fonctions réactives du point de vue chimique comme les fonctions amine et thiol éventuellement protégées. Si ces fonctions sont protégées, une étape de déprotection est nécessaire, ce qui complique l'étape de marquage. Si ces fonctions sont libres, une inhibition de la réaction enzymatique ou des réactions secondaires dans le cas de la synthèse chimique peuvent se produire.

Cette stabilité chimique de la fonction alkylcétone ne devrait pas en faire un bon candidat pour une réaction de couplage sur un marqueur et pourtant la présente invention démontre que, de manière surprenante, cette fonction est réactive pour le marquage et que, de plus, la détection du produit après marquage est très sensible.

Enfin, les nucléotides portant cette fonction présentent une neutralité excellente vis à vis des réactions enzymatiques puisque il est possible de remplacer complètement un nucléotide naturel par un nucléotide portant cette fonction alkylcétone dans une réaction enzymatique sans affecter le rendement de cette réaction et plus surprenant encore en l'améliorant dans certains cas.

Le document WO-A-95/24185 décrit un nucléoside modifié par un groupement alkylcétone dont la partie alkyle peut comporter jusqu'à 20 atomes de carbone. Ce composé est notamment destiné à la synthèse d'oligonucléotides, qui trouvent une application en thérapie, dans le diagnostic. L'introduction d'un groupement, notamment alkylcétone, sur le noyau pyrimidique du nucléoside décrit n'a pas vocation à fonctionnalisation en vue d'une réaction ultérieure dudit groupement, mais a pour but d'obtenir des analogues d'oligonucléotides qui présentent, par rapport aux oligonucléotides naturels, des propriétés intéressantes aux fins de leurs utilisations, telles qu'une plus grande facilité d'hybridation avec un acide nucléique cible, une plus grande résistance aux nucléases.

C'est l'objet de la présente invention de décrire un nouveau composé fonctionnalisé comportant une fonction alkylcétone et de formule (I) ci-dessous : 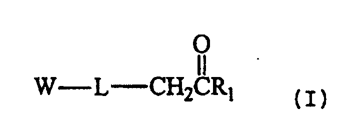 dans laquelle
- W représente un analogue nucléotidique ;
- L représente un bras de liaison entre W et le groupement alkylcétone, comportant au moins quatre atomes, avantageusement au moins huit atomes ; L est notamment choisi parmi les chaînes hydrocarbonées saturées ou insaturées, éventuellement interrompues par au moins une fonction amine, amide ou oxy ; de préférence, le bras de liaison comporte un enchaînement de 8 à 30 atomes ; en particulier, le bras de liaison comporte de 8 à 20 atomes dont au moins une fonction amide ;
- R₁ représente une chaîne alkyle, linéaire ou ramifiée, de préférence une chaîne alkyle ayant au plus 6 carbones ; avantageusement R₁ est un groupement méthyle.

Par analogue nucléotidique, on entend un nucléoside ou un nucléotide, un nucléoside ou un nucléotide portant une ou plusieurs modifications sur un des éléments constitutifs dudit nucléoside ou nucléotide comme une modification du sucre désoxyribose ou ribose comme le xylose, l'arabinose, les sucres de configuration alpha (FR 2 607 507), les PNA (M. Egholm et al., J. Am. Chem. Soc., (1992), 114, 1895-1897), les analogues de sucre comme le 4'-thio ribose ou désoxyribose, les sucres de configurations D ou L ; une modification de la base azotée ; une modification du phosphate ou de son équivalent pour les nucléotides ainsi que l'ensemble des groupements protecteurs utilisés dans la synthèse chimique.

Avantageusement, le composé répond à la formule
(I) dans laquelle W a la formule générale (II) : 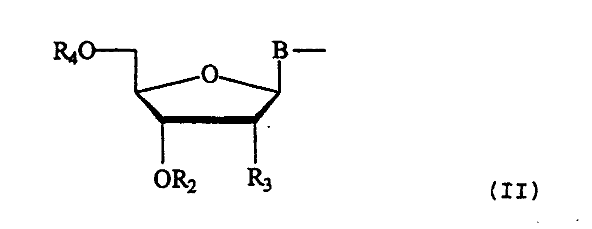
dans laquelle :
- R₂ représente H ou un groupement protecteur ;
- R₃ représente H, F, OH, SH, NH₂, OCH₃ ou OR₅ où R₅ représente un groupement protecteur ou une chaîne alkyle ;
- R₄ représente un radical H, un groupement protecteur ou un groupement mono, di ou triphosphate ;
- B représente une base azotée,
- W étant lié à L par l'intermédiaire de B.

La base azotée est choisie notamment parmi les purines ou pyrimidines comme l'adénine, la guanine, l'uracile, la cytosine, la thymine ou toute autre base modifiée permettant l'hybridation comme les bases modifiées naturelles (telles que la 6-céto-purine, la xanthine, la 5-méthyl-cytosine, la 2-amino-purine) ou non naturelles (telles que la thioguanine ou la 8-oxo-guanine, déazapurine, azapurine), ou des analogues de bases comme les bases universelles (telles que les dérivés nébularine, nitroindole ou nitropyrrole). Certaines fonctions des bases susceptibles d'interférer avec les stratégies de synthèse chimique en phase solide ou liquide peuvent être protégés par des groupements protecteurs appropriés.

Préférentiellement, la base azotée est l'adénine, l'uracile, la cytosine.

Le bras de liaison L est greffé sur une position quelconque de la base azotée ou de son analogue. De préférence le bras de liaison sera greffé sur une position ne perturbant pas l'hybridation. En particulier, le bras de liaison sera fixé sur l'amine en position 4 de la cytosine, la position 5 de l'uracile ou l'amine en position 6 de l'adénine.

Par groupement protecteur, on entend les groupements utilisés de manière classique dans la synthèse chimique de nucléosides, nucléotides et oligonucléotides (voir par exemple Chemistry of Nucléosides and Nucleotides, Edited by Leroy B. Townsend, Plenum Press, New York and London et Protocols for Oligonucleotides and Analogs, Synthesis and Properties, Edited by S. Agrawal, Humana Press, Totowa, New Jersey). De préférence dans le cas de la synthèse chimique, R₄ est un groupement 4,4'-diméthoxy trityle et R₂ est un groupement 2-cyanoéthyl-N,N-diisopropylphosphoramidite et R₃ est H ou OR₅, où R₅ est un groupement protecteur utilisé en synthèse d'oligoribonucléotide.

De préférence, dans le cas de la synthèse enzymatique, R₄ est un groupement triphosphate, R₂ est H est R₃ est OH.

Les groupements phosphate sont généralement sous forme de sels, et particulièrement des sels de lithium, de sodium, ou de triéthylammonium acétate.

L'invention concerne aussi un polynucléotide fonctionnalisé comprenant au moins un nucléotide fonctionnalisé tel que défini précédemment. Il peut être synthétisé par réaction chimique et/ou enzymatique. Dans le cas d'une synthèse par réaction enzymatique et notamment dans le cas d'une amplification enzymatique, la neutralité du nucléotide fonctionnalisé vis à vis des réactions enzymatiques permet l'incorporation de plusieurs nucléotides fonctionnalisés.

Par réaction enzymatique, on inclut toutes les réactions au cours desquelles intervient au moins une enzyme dont l'activité est liée à un nucléotide. Ainsi on entend toutes réactions comprenant au moins une étape enzymatique dans laquelle un nucléotide sert de substrat à l'enzyme, que ledit nucléotide soit transformé ou non au cours de cette étape enzymatique. A titre d'exemple de telles réactions sont choisies parmi celles utilisées dans les techniques de biologie moléculaire telles que la transcription, la ligation, l'élongation, la coupure et plus particulièrement dans les techniques d'amplification (voir par exemple l'article de E. Winn-Deen, Journal of Clinical assay, vol 19, p21-26, (1996)).

Ainsi, les enzymes dont les activités sont liées à des nucléotides peuvent en particulier être sélectionnées dans la liste non exhaustive suivante : les ADN polymérases ADN dépendantes telles que le fragment de Klenow de l'ADN polymérase I de *E. Coli*, la TAQ polymérase, les T7, T4 ou T5 ADN polymérases, les polymérases eucaryotes cellulaires ou virales, les ADN polymérases ARN dépendantes telles que les polymérases de AMV (Avian Myoblastosis Virus), de MMLV (Moloney Murine Leukemia Virus) ; les ARN polymérases telles que les T7, T3 SP6, N4, PBSII ARN polymérases, l'ARN polymérases de *E. Coli* ; les enzymes à activité nucléasiques telles que les endonucléases de restriction, la Rnase H ; ou encore les polyA polymérases, les réplicases comme la Q-béta-réplicase, les terminal transférases ou les ligases.

Les enzymes thermostables présentant les activités enzymatiques décrites ci-dessus sont aussi utilisables dans l'invention.

Selon un mode préféré, on choisira pour la synthèse du polynucléotide fonctionnalisé les techniques utilisant une étape de transcription comme la NASBA (Nucleic Acid Sequence Based Amplification), la TMA (Transcription Mediated Amplification) ou une transcription post PCR (Polymerase Chain Reaction) comme décrit dans les articles de R.J. Lipshutz et al, Biotechniques, 19(3), p442-447, 1995 ou M. Kozal et al, Nature Médecine , 2(7), p753-759, 1996.

Les éléments et conditions nécessaires à la mise en oeuvre de ces réactions enzymatiques pour obtenir un polynucléotide sont bien connus de l'homme de métier. L'ouvrage Current Protocols in Molecular Biology, édité par F.M. Ausubel, R. Brent, R.E. Kingston, D.D. Moore J.G. Seidman, J.A. Smith et K. Struhl, John Wiley & Sons, 1996 volume 1 chapitre 3, indique les méthodes de manipulation enzymatique de l'ADN et l'ARN. De même, l'ouvrage « Molecular Methods for Virus Detection » édité par D.L. Wiedbrand et D.H. Farkas, Academic press, San Diego, 1995 notamment dans les chapitres 8, 9, 12, 13, 14, 15 et 16 donne des exemples pour les techniques d'amplification enzymatique.

Par synthèse chimique, on entend toutes les méthodes aussi bien en phase solide qu'en phase liquide dans lesquelles un monomère nucléotidique convenablement protégé réagit avec un autre monomère ou polymère nucléotidique par une réaction de couplage.

Les méthodes de synthèse chimique sont données par exemple dans « Methods in Molecular Biology, volume 20, Protocols for oligonucleotides and analogs », édité par S. Agrawal, Humana Press, Totowa, New Jersey, 1993 et « Methods in Molecular Biology, volume 26, Protocols for oligonucleotides conjugates », édité par S. Agrawal, Humana Press, Totowa, New Jersey, 1994.

Par polynucléotide, on entend un enchaînement d'au moins 2 monomères nucléotidiques. De préférence, si le polynucléotide est synthétisé par voie chimique, la taille est inférieure à 300 nucléotides et avantageusement à 150. De préférence, si le polynucléotide est synthétisé par réaction enzymatique, la taille est inférieure à 20 kb et avantageusement inférieure à 10 kb.

Les deux voies de synthèse chimique et enzymatique peuvent être combinées pour préparer un polynucléotide .

L'invention concerne aussi un polynucléotide fonctionnalisé marqué comprenant au moins un composé fonctionnalisé de formule générale (I') : 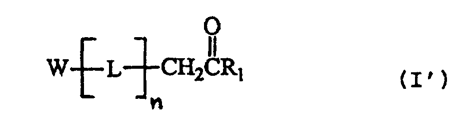 dans laquelle
W représente un analogue nucléotidique, tel que défini précédemment,
L représente un bras de liaison comportant au moins quatre atomes,
n représente un indice égal à 0 ou 1,
R₁ représente une chaîne alkyle, linéaire ou ramifiée,
le groupement alkylcétone dudit composé fonctionnalisé ayant interagi avec un réactif de marquage.

W, L et R₁ répondent avantageusement aux définitions données ci-dessus pour décrire des composés fonctionnalisés préférés de l'invention.

Par réactif de marquage, on entend un traceur générant directement ou indirectement un signal détectable et susceptible de réagir avec la fonction alkylcétone.

Une liste non limitative de ces traceurs suit :
- les enzymes qui produisent un signal détectable par exemple par colorimétrie, fluorescence, luminescence, comme la peroxydase de raifort, la phosphatase alcaline, la béta galactosidase, la glucose-6-phosphate déshydrogénase ;
- les chromophores comme les composés fluorescents, luminescents, colorants ;
- les groupements à densité électronique détectable par microscopie électronique ou par leur propriété électrique comme la conductivité, l'ampérométrie, la voltamétrie, les mesures d'impédance ;
- les groupements détectables par des méthodes optiques comme la diffraction, la résonance plasmon de surface, la variation d'angle de contact ou physiques comme la spectroscopie de force atomique, l'effet tunnel ;
- les molécules radioactives comme le ³²P, le ³⁵S
ou le ¹²⁵I.

De préférence, le traceur est un composé fluorescent de faible encombrement stérique comme la fluorescéine, le dansyle, les chromophores du type IR (Li-COR Inc, Lincoln NE, USA), CY5 et CY3 (Randolph J.B. and al, Nucleic Acids Res., 25(14), p2923-2929, 1997) et leurs dérivés. Par faible encombrement stérique, on entend un poids moléculaire inférieur à 1000 g/mole.

Pour réagir avec la fonction alkylcétone, ce réactif de marquage doit porter une fonction nucléophile susceptible de réagir avec une fonction alkylcétone comme les fonctions alcoxyamine ou hydrazine.

De préférence, la fonction choisie est l'alcoxyamine qui peut être introduite par tous moyens directs ou indirects. Par moyen direct on entend une liaison covalente entre le traceur ou une molécule portant le traceur et la fonction alcoxyamine. Par moyen indirect, on entend les systèmes de complexation du type métal/chélate ou les systèmes d'affinité c'est à dire les haptènes détectables par un anticorps spécifique ou une protéine comme le couple biotine/avidine ou streptavidine, sucre/lectine. Dans ce cas c'est l'anticorps ou la protéine qui porte le traceur et c'est l'haptène qui porte la fonction alcoxyamine.

En particulier le réactif de marquage est de formule :

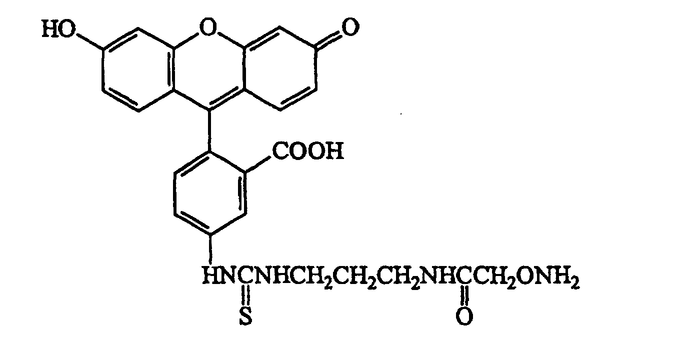

L'invention concerne également un support solide sur lequel est fixé par covalence un nucléotide, un nucléoside ou un polynucléotide selon l'invention.

Pour réaliser cette fixation, l'on fait réagir un nucléotide, un nucléoside ou un polynucléotide comportant un groupement alkylcétone avec un support solide sur lequel est présent une fonction alcoxyamine ou hydrazine, préférentiellement alcoxyamine.

Dans un premier mode de réalisation, le polynucléotide est préformé et la réaction finale consiste à greffer, à une position prédéterminée sur le support, le polynucléotide. Dans un mode particulier de réalisation, les polynucléotides sont des oligonucléotides de synthèse (par voie chimique) de taille courte (inférieure à 50 bases), dans un deuxième mode particulier, les polynucléotides sont de taille supérieure à 50 bases et sont préparés par des méthodes enzymatiques telles que l'amplification enzymatique.

Dans un deuxième mode de réalisation, le nucléoside ou nucléotide est additionné par étapes successives (allongement de chaîne) sur le support pour obtenir à la fin du cycle de synthèse un polynucléotide greffé à une position prédéterminée sur le support solide.

Une application préférentielle de ces supports greffés est d'obtenir des biopuces pour l'analyse de gènes.

A titre d'illustration, des exemples de ces biopuces sont donnés dans les publications de G. Ramsay, Nature Biotechnology, 16, p40-44, 1998 ; F. Ginot, Human Mutation, 10, p1-10, 1997 ; J. Cheng et al, Molecular diagnosis, 1(3), p183-200, 1996 ; T. Livache et al, Nucleic Acids Research, 22(15), p2915-2921, 1994 ; J. Cheng et al, Nature Biotechnology, 16, p541-546, 1998 ou dans les brevets US-A-4 981 783 (Augenlicht), US-A-5 700 637 (Southern), US-A-5 445 934 (Fodor), US-A-5 744 305 (Fodor), US-A-5 807 522 (Brown).

L'invention concerne aussi un procédé de détection d'un acide nucléique cible dans un échantillon dans lequel on met en contact cet acide nucléique cible éventuellement prétraité avec au moins un composé fonctionnalisé répondant à la formule (I'), en présence des éléments et dans des conditions nécessaires à l'obtention d'un polynucléotide de l'invention, pour obtenir un polynucléotide fonctionnalisé ; à marquer ledit polynucléotide avec un réactif de marquage puis à détecter ledit polynucléotide marqué. Les éléments et conditions précités sont bien connus de l'homme du métier.

Par prétraitement, on entend les différentes étapes de traitement de l'échantillon pour rendre accessible l'acide nucléique cible, comme par exemple, la lyse, la fluidification, la concentration.

De préférence le polynucléotide fonctionnalisé est obtenu par une réaction d'amplification enzymatique qui agit sur l'acide nucléique cible qui sert de matrice et qui est capable d'incorporer le nucléotide fonctionnalisé.

Avantageusement la technique d'amplification enzymatique est la NASBA (Nucleic Acid Sequence Based Amplification), la TMA (Transcription Mediated Amplification) ou une transcription post PCR (Polymerase Chain Réaction) comme décrit dans les articles de R.J. Lipshutz et al, Biotechniques, 19(3), p442-447, 1995 ou M. Kozal et al, Nature Médecine , 2(7), p753-759, 1996.

Le polynucléotide marqué peut être détecté qualitativement et/ou quantitativement en phase homogène ou hétérogène. Un mode préférentiel de détection consiste à fixer le polynucléotide marqué sur un support solide par l'intermédiaire d'une réaction d'hybridation entre le polynucléotide marqué et un autre polynucléotide lui même fixé sur le support solide puis à révéler la présence du polynucléotide marqué après une étape de lavage. Cette révélation se fait directement par lecture comme par exemple avec un scanner ou une caméra si le traceur est une molécule fluorescente.

Le procédé de détection est particulièrement utile dans le cas où une multitude de polynucléotides sont fixés sur le support solide à une position prédéterminée pour former une « puce à ADN ».

En effet, la densité des polynucléotides fixés sur le support solide imposent des contraintes stériques importantes lors de l'hybridation et le polynucléotide marqué selon l'invention permet une bonne sensibilité de détection. Des exemples de ces puces sont donnés par exemple cités dans les publications et brevets mentionnés précédemment.

Le procédé de détection est applicable pour le séquençage, le profil d'expression des ARN messagers ou le criblage de mutation, le diagnostic de maladies infectieuses ou génétiques.

Une étape de fragmentation peut avoir lieu pour favoriser l'hybridation du polynucléotide marqué sur la puce à ADN, avant, conjointement ou après l'étape de marquage.

L'invention concerne en outre un procédé de détection d'un acide nucléique cible dans un échantillon dans lequel on met en contact cet acide nucléique cible avec un polynucléotide fonctionnalisé de l'invention, on fait réagir le réactif de marquage et l'on détecte la présence de l'acide nucléique cible.

Un autre moyen de pratiquer l'invention est de faire réagir, avant l'hybridation avec l'acide nucléique cible, le polynucléotide fonctionnalisé et le réactif de marquage. Cet acide nucléique cible peut avoir été amplifié par une technique d'amplification enzymatique.

Enfin, l'invention concerne le procédé de détection d'un acide nucléique cible selon lequel on dispose d'un polynucléotide marqué de l'invention, on met en contact l'acide nucléique avec le polynucléotide marqué et on détecte la présence de l'acide nucléique cible.

Le terme « support solide» tel qu'utilisé ici inclut tous les matériaux sur lesquels peut être immobilisé un polynucléotide pour une utilisation dans des tests diagnostiques et dans des processus de séparation. Des matériaux naturels ou de synthèse, modifiés ou non chimiquement peuvent être utilisés comme support solide, notamment les polysaccharides tels que les matériaux à base de cellulose, par exemple du papier, des dérivés de cellulose tels que l'acétate de cellulose et la nitrocellulose, du dextran ; des polymères tels que polychlorures de vinyle, polyéthylènes, polystyrènes, polyacrylates, polyamides, ou des copolymères à base de monomères du type styrène, esters d'acides carboxyliques insaturés, chlorure de vinylidène, diènes ou composés présentant des fonctions nitrile (comme l'acrylonitrile) ; des copolymères chlorure de vinyle/propylène, chlorure de vinyle/acétate de vinyle ; des fibres naturelles telles que le coton et des fibres synthétiques telles que le nylon ; des matériaux inorganiques tels que la silice, le quartz des verres, des céramiques ; des latex, c'est-à-dire des dispersions aqueuses colloïdales d'un polymère quelconque insoluble dans l'eau; des particules magnétiques ; des dérivés métalliques, des gels etc.

Les exemples qui suivent permettent d'illustrer quelques avantages de l'invention sans toutefois en limiter la portée.

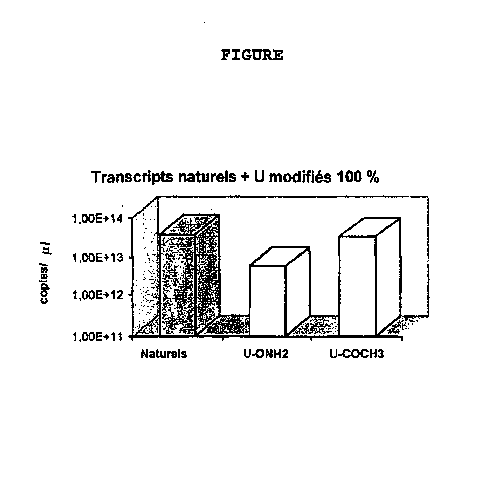

La Figure annexée représente les rendements de transcription mesurés en fonction du nucléotide utilisé.

### EXEMPLE I : SYNTHESE DE NUCLEOTIDES METHYLCETONES

### I.1. Synthèse de l'Uridine(C5)-C9-méthylcétone 1

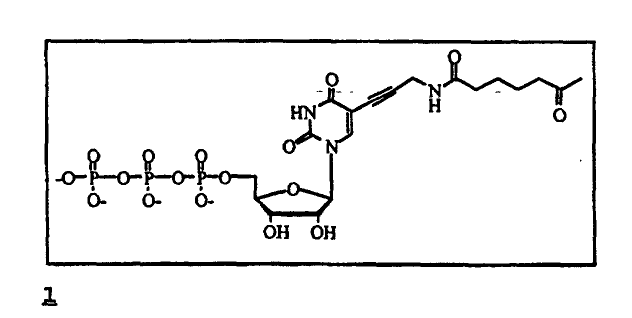

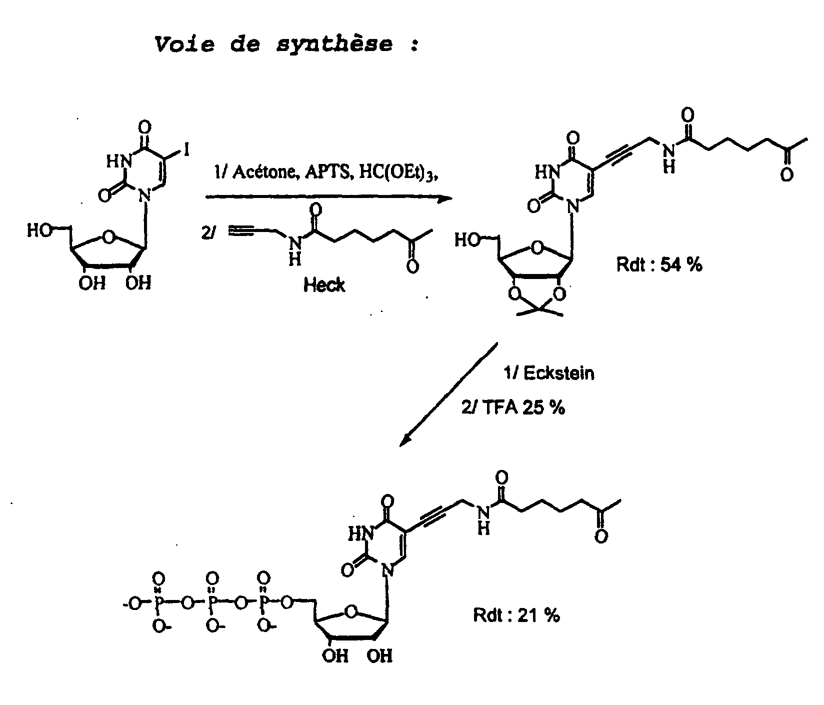

La protection des positions 2',3'-OH est réalisée par action de l'acétone en milieu acide selon le protocole décrit dans "Nucleic acid chemistry, Editors Townsend-Tipson, Wiley-Interscience, John Wiley & Sons, p 765-766 (1979)".

### Synthèse de la chaîne méthylcétone

L'introduction du motif méthylcétone sur le bras se fait par couplage peptidique entre la propargylamine et l'acide 6-oxo-heptanoïque.

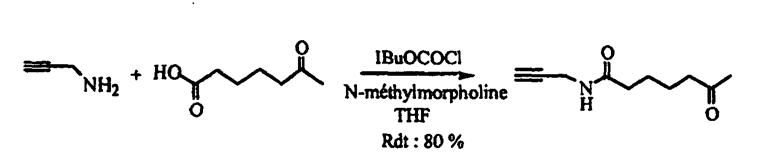

On solubilise l'acide 6-oxo-heptanoïque (2,75 g, 18,16 mmol) dans 40 ml de THF anhydre. La solution est placée à 0°C sous atmosphère d'argon. On ajoute alors la N-méthylmorpholine (2ml, 18,16 mmol) puis la propargylamine (1,25 ml, 18,16 mmol) après 15 min et on laisse revenir à température ambiante. Après 30 minutes de réaction, on filtre le précipité et on évapore à sec.

Le résidu obtenu est repris dans du dichlorométhane et est lavé avec une solution aqueuse de soude 0,1N puis avec une solution aqueuse de l'acide chlorhydrique 1N et enfin avec de l'eau saturée en chlorure de sodium.

Après séchage sur Na₂SO₄, le dichlorométhane est évaporé et le résidu est chromatographié sur gel de silice (éluant : acétate d'éthyle). On obtient ainsi le produit sous forme d'une poudre blanche (2,6 g, 14,4 mmol ; 80 %).

La chaîne méthylcétone a été caractérisée par RMN du proton, RMN du Carbone 13 et par spectrométrie de masse.

### Introduction de la chaîne méthylcétone sur le nucléoaide : couplage de Heck (Hobbs, J. Org. Chem., 1989, 54, 3420-3422)

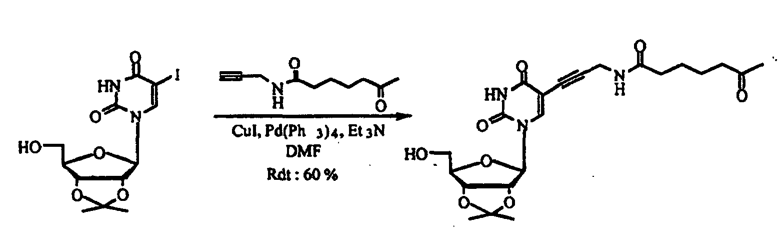

A 5 ml de DMF dégazés et placés sous argon, on ajoute de la 5-iodouridine 2',3'-isopropylidène (500 mg ; 1,22 mmol) et de l'iodure de cuivre (44 mg, 0,232 mmol). La réaction est placée à l'obscurité puis on ajoute de la triéthylamine (323 µl, 2,32 mmol) et la chaîne contenant la fonction méthylcétone (630 mg, 3,48 mmol).

Le mélange est laissé sous argon pendant 10 minutes. On ajoute alors le tétrakis-triphénylphosphine palladium (134 mg, 0,116 mmol). Après 3 heures de réaction, on évapore la DMF et on coévapore avec de l'acétonitrile. Le résidu est repris dans de l'acétate d'éthyle et la phase organique est lavée avec une solution aqueuse saturée en chlorure de sodium. Après séchage sur Na₂SO₄ et évaporation, le résidu est chromatographié sur gel de silice (éluant : acétate d'éthyle/méthanol : 90/10).

Après évaporation on obtient le nucléoside méthylcétone sous forme d'une poudre blanchâtre (340 mg, 0,73 mmol, 60 %). Le produit a été caractérisé par RMN du proton, du Carbone 13 et par spectrométrie de masse. On obtient ainsi le nucléoside méthylcétone correctement protégé pour l'introduction du triphosphate en 5'.

### Obtention du nucléoside uridine(C5)-C9-méthylcétone triphosphate 1

*Phosphorylation :* Eckstein, J. *Org.* Chem., ***1989**, 54*, *631-635*

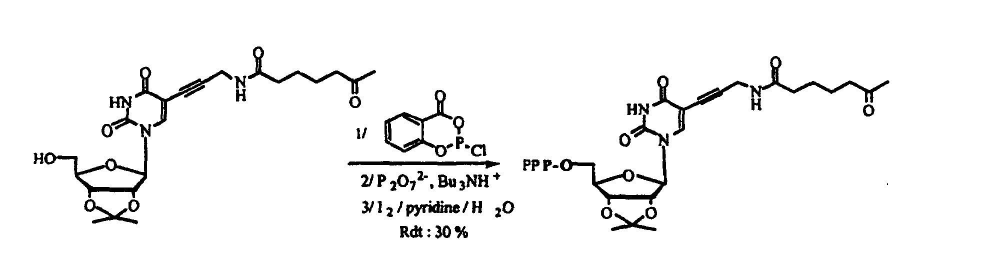

Le nucléoside méthylcétone (46 mg, 0,1 mmol) est dissous dans de la pyridine anhydre et est évaporé 2 fois. On ajoute alors sous argon 100 ml de pyridine, 300 ml de dioxane et une solution fraîchement préparée de 2-chloro-4H-1,2,3-dioxaphosphorin-4-one (1 M) dans du dioxane (130 µl ; 130 µmol), on laisse l'agitation pendant 20 minutes puis on ajoute une solution 0,5 M de pyrophosphate de tributylamonnimum dans de la DMF anhydre (320 µl, 0,16 mmol) et simultanément 130 µl de tributylamine. Après 30 minutes, on ajoute 2 ml d'une solution d'iode à 1 % dans un mélange pyridine / eau (98/2 : v/v).

Après 20 minutes d'agitation on détruit l'excès d'iode avec une solution aqueuse de NaHS03 à 5 % et on maintient l'agitation pendant 10 minutes. On évapore à sec et on effectue une extraction avec un mélange eau/dichlorométhane. La phase aqueuse est évaporée puis on effectue une chromatographie (flash) en phase inverse C18 (éluant : eau/méthanol 1/1). Les fractions contenant le produit sont évaporées et l'échange de contre ion est réalisé par passage sur résine Dowex,Na+. On récupère ainsi le triphosphate protégé (0,03 mmol ; 30%).

### Déprotection

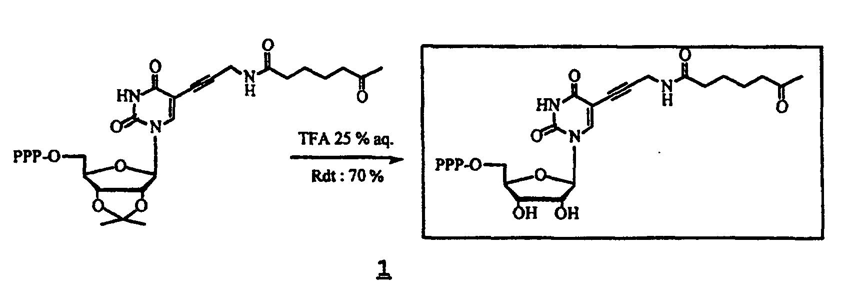

Le triphosphate protégé (0,03 mmol) est repris dans 15 ml d'eau milli-Q auxquels 15 ml d'une solution aqueuse de TFA à 25 % sont ajoutés. La solution est agitée pendant 15 minutes puis on évapore et coévapore 2 fois avec de l'eau.

On reprend dans 10 ml d'eau milli-Q et on neutralise avec de la soude 0,1 N jusqu'à pH 8. Après l'évaporation Le triphosphate protégé est purifié sur phase inverse C18 (H₂O puis H₂O/MeOH, 1/1). L'échange de contre-ion est réalisé par passage sur résine échangeuse de cations (Dowex Na⁺). Les fractions contenant le produit sont évaporées et dosées. On récupère ainsi 0,021 mmol (70 %) du nucléoside uridine(C5)-C9-méthylcétone triphosphate 1 qui a été caractérisé par RMN du proton, RMN du carbone 13, et par RMN du phosphore 31.

### I.2. Synthèse de l'Adénosine(N6)-C10-méthylcétone 2

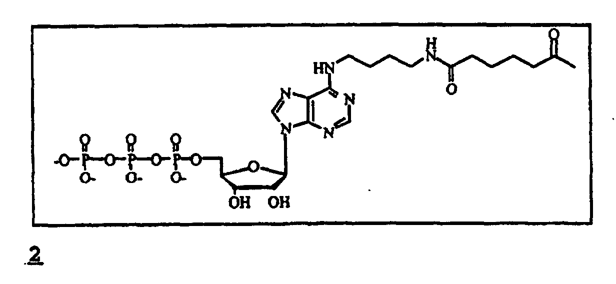

### Voie de synthèse

L'introduction du diaminobutane en position 6 de l'adénosine est réalisée par substitution du groupement triazolo porté par le nucléoside intermédiaire protégé. La fonction méthylcétone est ensuite introduite par couplage peptidique au niveau nucléosidique. Après déprotection du groupement silyle en 5', la phosphorylation est effectuée par la méthode de Eckstein. Après déprotection on obtient le triphosphate attendu, caractérisé par RMN du proton, du phosphore 31.

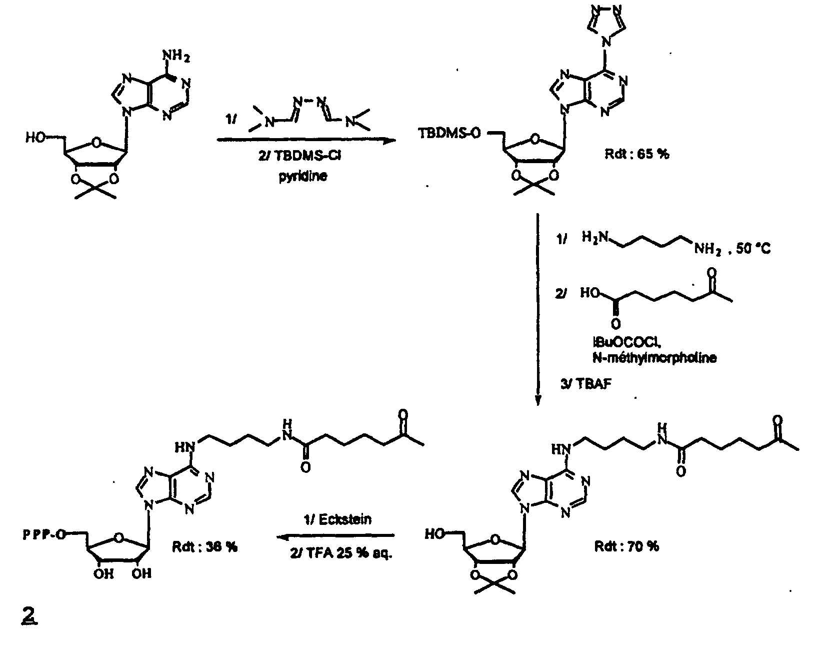

### Synthèse du nucléoside triazolo protégé

### Préparation de l'adénosine 2', 3'-isopropylydène

### (Nucleic Acid Chemistry, part 2, Editors Townsend, Tipson, Wiley Interscience, John Wiley & Sons p. 768, (1978))

A une suspension d'adénosine (5 g, 18,7 mmol) dans de l'acétone (10 ml) contenant de l'APTS (acide paratoluène sulfonique) (3,9 g, 20,6 mmol), on ajoute goutte à goutte sous argon de l'orthoformate d'éthyle (12,44 ml, 74,8 mmol). Après une nuit de réaction, on ajoute 110 ml d'eau contenant 1,86 ml d'ammoniaque à 27 %. Après 30 minutes d'agitation, le milieu réactionnel est évaporé jusqu'à apparition de cristaux blancs. Après 12 h à +4°C, on obtient un précipité blanc que l'on recristallise dans l'eau. On obtient 4,17 g (13,5 mmol, 72 %) de produit sous forme d'une poudre blanche. Cet intermédiaire a été caractérisé par RMN du proton.

### Synthèse de l'amidine (Bartlett et Humphreg, J. Chem. Soc., 1967, 1664-1666)

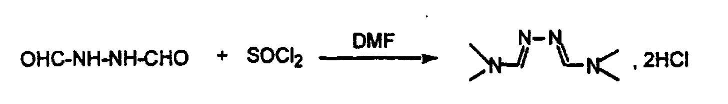

Le chlorure de thionyle (39,96 g, 24,5 ml, 0,338 mol) est ajouté goutte à goutte au N-N'-diformylhydrazine (12 g, 0,136 mol) dans le DMF (270 ml) à 10°C. Le mélange devient jaune. On maintient l'agitation pendant 2 jours. Le précipité obtenu est filtré et lavé par de la DMF puis par de l'éther. Après séchage sous vide, on obtient l'amidine avec un rendement de 95 % (28 g, 0,130 mol).

### Préparation de l'intermédiaire triazolo (Samano, Miles, Robins, J. Am. Chem. Soc., 1994, 116, 9331-9332)

L'adénosine-isopropylidène (1 g, 3,2 mmol) et l'amidine décrite ci-dessus (1,4 g, 6,5 mmol) sont agitées dans de la pyridine (15 ml) à 100°C sous argon pendant 48 h. La pyridine est alors évaporée et coévaporée avec du toluène. L'huile obtenue est ensuite reprise par de l'acétate d'éthyle et cette phase organique est lavée avec de l'eau saturée en NaCl. Après séchage sur Na₂SO₄ et évaporation, on obtient le nucléoside triazolo sous forme d'une poudre blanche avec un rendement de 60 % (700 mg, 1,9 mmol). Il a été ensuite caractérisé par RMN du proton.

### Protection en 5'-du dérivé triazolo

On solubilise le nucléoside triazolo (1 g, 2,8 mmol) dans 20 ml de pyridine. On ajoute à 0°C sous argon le (TBDMS - Cl, Chlorure de terbutyldiméthylsilyle), (462 mg, 3 mmol). On maintient l'agitation pendant deux heures puis on évapore la pyridine. Le résidu ainsi obtenu est chromatographié sur gel de silice (éluant : CH₂Cl₂:/Méthanol : 95/5). Après évaporation, on obtient l'adénosine intermédiaire totalement protégé sous forme d'une poudre blanche (1,25 mg, 2,6 mmol, 93 %). Ce nucléoside a été caractérisé par RMN du proton, du carbone 13 et par spectrométrie de masse.

### Introduction de la chaîne diaminobutane sur l'adénosine intermédiaire protégé

L'adénosine triazolo (1,25 mg, 2,64 mmol) est solubilisé dans 10 ml d'acétonitrile. On ajoute alors la diaminobutane (2,7 ml, 25,4 mmol) et on agite à 50°C sous argon. Après 5 heures, on évapore le solvant et on reprend le résidu dans de l'acétate d'éthyle. On lave cette phase organique avec de l'eau saturée en NaCl. Après séchage sur Na2SO4 et évaporation en chromatographie sur gel de silice (éluant : CH₂Cl₂ / MeOH : 8/2, puis CH₂Cl₂ / MeOH : 8/2 en présence de 2% d'ammoniaque).

Après évaporation, on obtient le produit sous forme d'une huile (1 g, 2 mmol, 80%).

Le nucléoside aminé a été caractérisé par RMN du proton, du carbone 13 et par spectrométrie de masse.

### Couplage à la chaîne méthylcétone et déprotection en 5'

L'acide 6-oxo-heptanoïque (288 mg, 2 mmol) est dissous dans 5 ml de THF anhydre. On place la solution à 0°C sous argon. On ajoute alors la N-méthylmorpholine (223 µl, 2 mmol) et après 5 min, le dichloroformiate d'isobutyle (258 µl, 2 mmol). Après 15 minutes, on rajoute le nucléoside aminé. Après 2 heures, on évapore le THF et on reprend le résidu par de l'éther. On lave avec une solution aqueuse de NaOH 1N puis avec de l'eau saturée en NaCl. Après séchage sur Na₂SO₄ et évaporation on obtient une huile. On effectue la désilylation en reprenant l'huile dans 10ml de THF auxquels on ajoute du TBAF (3,25 ml d'une solution de 1M dans THF). Après une heure on évapore le solvant. On solubilise le résidu dans du dichlorométhane et on effectue un lavage avec de l'eau saturée en NaCl. Après séchage et évaporation, le résidu est chromatographié sur gel de silice (éluant : CH₂Cl₂, puis CH₂Cl₂ / MeOH : 95/5).

Après évaporation on obtient 870 mg du nucléoside portant la fonction méthylcétone et déprotégé en 5' (1,7 mmol, 85 % sur 2 étapes). Il est caractérisé par RMN du proton, du carbone 13 et par spectrométrie de masse.

### Phosphorylation et obtention du nucléotide Adénosine-(N6)-C10-méthylcétone 2

L'adénosine-(N6)-C10-méthylcétone protégé en 2', 3' par l'isopropylidène est dissous dans de la pyridine anhydre et est évaporée deux fois. On ajoute alors sous argon 500 µl de pyridine, 1,5 ml de dioxane et une solution fraîchement préparée de 2-chloro 4H-1,2,3-dioxaphosphain-4-one (1 M) dans le dioxane (650 µl, 0,65 mmol). On laisse l'agitation pendant 20 minutes puis on ajoute une solution 0,5 M de pyrophosphate de tributylamonium dans de la DMF anhydre (1,6 ml, 0,8 mmol) et simultanément 650 µl de tributylamine.

Après 30 minutes, on ajoute 10 ml d'une solution d'iode à 1% dans un mélange pyridine/eau (98/2, v/v). Après 20 minutes, on détruit l'excès d'iode avec une solution aqueuse de NaHSO₃ à 5% et on maintient l'agitation pendant 10 minutes on évapore à sec et on effectue une extraction eau/dichlorométhane. La phase aqueuse est évaporée puis on effectue une purification en phase inverse (C18), (éluant : H₂O/MeOH). Les fractions contenant le produit sont évaporées et l'échange de contre ion est réalisé par passage sur résine Dowex, Na+. On récupère ainsi le triphosphate protégé (0,28 mmol, 56 %).

### Déprotection

On reprend 0,035 mmol de triphosphate protégé dans 17,5 ml d'eau milli-Q auxquels on rajoute 17,5 ml d'une solution aqueuse de TFA à 25%. On agite pendant 15 minutes puis on évapore et coévapore deux fois avec de l'eau, on reprend dans 10 ml d'eau milli-Q et on neutralise avec de la soude 0.1 M jusqu'à pH 8. Après évaporation, on effectue une purification en C18 (éluant: H2O ; H2O / MeOH). Les fractions contenant le produit sont évaporées et dosées. On obtient ainsi 0,022 mmol (63%) de l'adénosine-(N6)-C10-méthylcétone triphosphate 2.

### I.3. Synthèse de la cytidine (N4)-C10-méthycétone 3

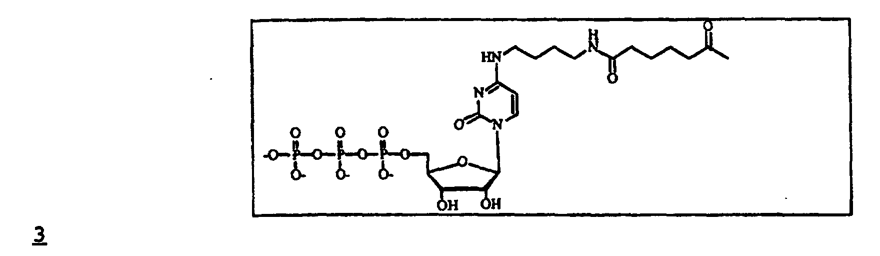

Ce nucléotide a été préparé de deux façons :
- *Voie nucléotidique* : couplage entre un ester activé de la chaîne méthylcétone et la cytidine triphosphate aminé. 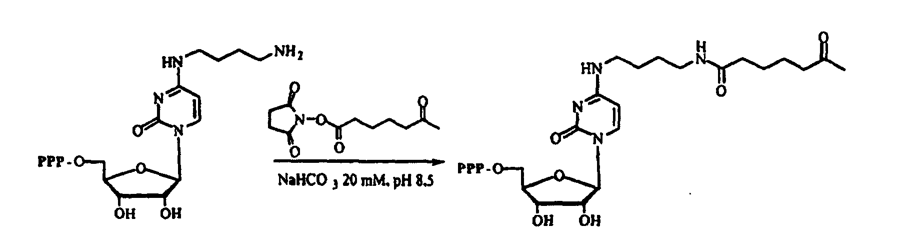
- *Voie nucléosidique* : synthèse du nucléoside méthylcétone puis phosphorylation par la méthode de Eckstein.

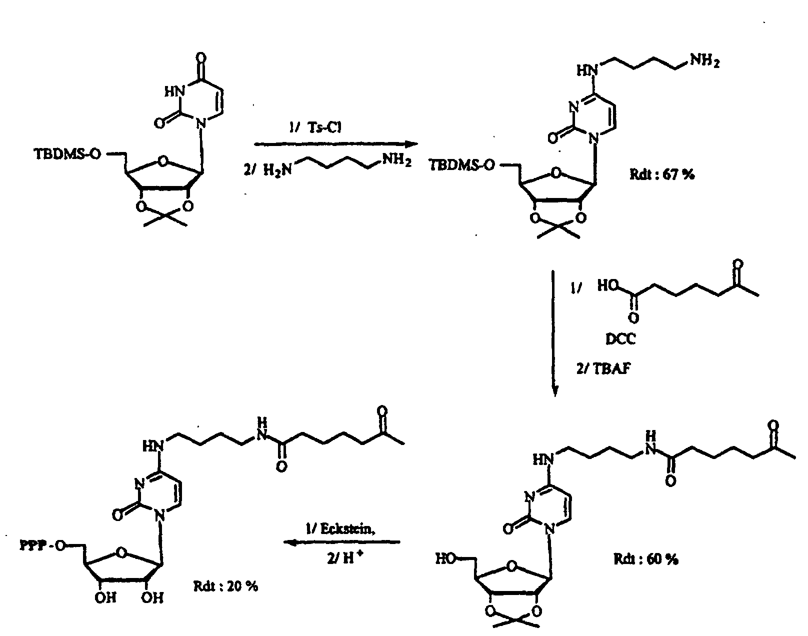

### EXEMPLE II : SYNTHESE DU FLUOROPHORE OXYAMINE 4

### II. 1. Schémas de synthèse

L'introduction de la chaîne oxyamine sur la fluorescéine se fait en trois étapes : la première étape est une addition nucléophile du 1,3-diaminopropane sur l'isothiocyanate de fluorescéine (FITC). Après purification en phase inverse, le motif oxyamine protégé sous forme de Fmoc est introduit par couplage peptidique. L'oxyamine libre est générée par déprotection en milieu basique.

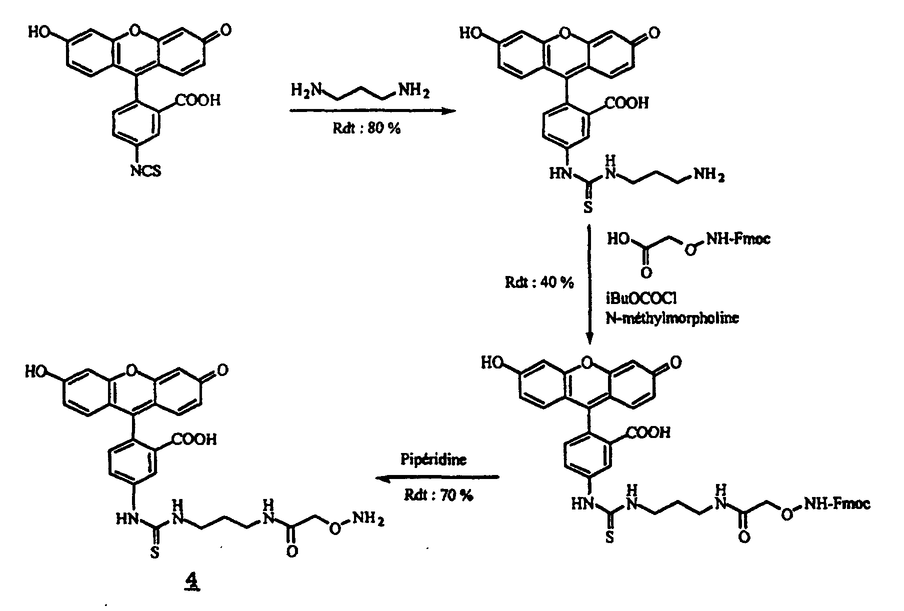

### Introduction de la chaîne 1,3-diaminopropane :

On ajoute à 20 ml de DMF anhydre de la diaminopropane (585 µl, 6,96 mmol). On additionne alors, sous argon, goutte à goutte de l'isothiocyanate de fluorescéine (FITC), (500 mg, 1,16 mmol) solubilisé dans 7 ml de DMF anhydre. On maintient l'agitation pendant 15 minutes après la fin de l'addition. On évapore à sec et on coévapore deux fois avec de l'eau. Le résidu est chromatographié en phase inverse (C18) : (Eluant : H₂O / MeOH : 1/1).

On récupère ainsi le produit sous forme d'une poudre orange (420 mg, 0,93 mmol, 80 %). Il est caractérisé par RMN du proton, du Carbone 13 et par spectrométrie de masse.

### Introduction du groupement alcoxyamine protégé :

On solubilise la carboxyalcoxyamine protégée par le Fmoc : HOOC - CH₂ - ONH -Fmoc (473 mg, 1,5 mmol dans 10 ml de DMF anhydre). On place à 0°C sous argon. On ajoute alors la N-méthylmorpholine (166 µl, 1,5 mmol) et après 15 minutes on ajoute la fluorescéine portant la chaîne diaminopropane (350 mg, 0,75 mmol). Après 1 heure de réaction, on évapore à sec la DMF. Le résidu obtenu est chromatographié sur gel de silice (CH₂Cl₂ / MeOH : 85/15 (dépôt solide). On obtient ainsi la fluorescéine-alcoxyamine protégée sous forme d'une poudre orange (227 mg, 0,3 mmol, 40 %). Il est caractérisé par RMN du proton, du Carbone 13 et par spectrométrie de masse.

### Obtention du marqueur fluorescéine-alcoxyamine 4 :

On solubilise la fluorescéine protégée par le fmoc (100 mg, 0,13 mmol) dans 2 ml de DMF anhydre. On ajoute alors de la pyridine (20 µl, 0,2 mmol). Après 15 minutes, on évapore à sec puis on effectue une purification en phase inverse C18 (éluant : H₂O /CH₃CN : 1/1 puis CH₃CN). Après évaporation, on obtient le produit sous forme d'une poudre orange (49 mg, 0,09 mmol, 70%). Ce fluorophore a été caractérisé par RMN du proton, RMN 13C et par spectrométrie de masse.

### EXEMPLE III : REACTIVITE DES COMPOSES METHYLCETONE AVEC LE FLUOROPHORE

La réactivité a été testée au niveau nucléosidique et au niveau nucléotidique :

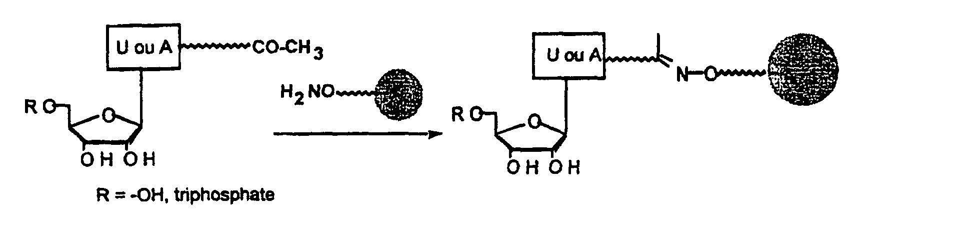

La réaction est réalisée en présence de 1,1 eq. de fluorophore-ONH₂ (4) par rapport au composé méthylcétone. La réaction est rapide et sélective. Au niveau nucléosidique, les adduits ont été caractérisés par RMN du proton et par spectrométrie de masse.

### EXEMPLE IV : INCORPORATION DES URIDINES-METHYLCETONE ET MARQUAGE POST- TRANSCRIPTION

### IV.1. Description des étapes principales Transcriptions

Les transcriptions ont été réalisées à partir de cible PCR (fragment de l'ARN 16 S de *Mycobacterium Tuberculosis* (Mtb) (Troesch A. et al, J. Clin. Microbiol., 37(1), 49-55, 1999) ou fragment de la reverse transcriptase de HIV (Kozal M.J. et al, Nature Médecine, 2(7), 753-759, 1996) en utilisant la T7 RNA polymérase et différents rapports entre le nucléotide fonctionnalisé et les nucléotides naturels, tout en gardant à 1mM la concentration totale de chaque nucléotide. Ce rapport entre le nucléotide fonctionnalisé et le nucléotide naturel correspondant est exprimé en pourcentage et les rapports utilisés sont en général 0, 30, 70 et 100 %. Le point à 0 % sert de témoin de transcription, puisque dans ce cas il n'y a pas de nucléotide fonctionnalisé et la réaction de transcription comporte les 4 nucléotides naturels. Dans le cas d'un rapport à 100% , cela signifie que le nucléotide fonctionnalisé représente 100% du nucléotide étudié (les trois autres nucléotides nécessaires à la réaction de transcription étant naturellement les nucléotides naturels). Ce rapport de 100% pour un nucléotide fonctionnalisé est le test le plus significatif de la neutralité vis-à-vis d'une réaction enzymatique puisque l'enzyme doit incorporer ce nucléotide pour fonctionner correctement. Le temps d'incubation de la réaction de transcription est de 1h à 42°C.

Les transcriptions sont analysées par électrophorèse sur gel de polyacrylamide en conditions dénaturantes (6% acrylamide, 7M urée, 1XTBE). Le volume déposé est de 5 µl et la migration s'effectue pendant 45 min à 150 V. La visualisation des transcripts, naturels ou fonctionnalisés avec la fonction méthylcétone est réalisée sous lampe UV après coloration au bromure d'éthidium.

### Dosage

La quantité de transcrits produits dans chaque réaction est déterminée par dosage UV après purification d'un aliquote issu d'une réaction de transcription.

### Digestion enzymatique

Les transcrits sont purifiés sur des filtres microcon-50 (Amicon, Beverly, MA) pour éliminer l'excès de nucléotides non incorporés. Ils sont ensuite hydrolysés selon le protocole décrit dans la demande de brevet WO98/05766 en utilisant la nucléase P1 (Boehringer référence 2362251,2U, 2h à 37°C) et la phosphatase alcaline (Boehringer-Mannhein référence 713023,1U, 1h à 37°C). Les digestions sont menées sur 4.10¹⁴ copies de transcrits. La composition en nucléosides est déterminée par CLHP en phase inverse, par comparaison avec des standards nucléosidiques composés d'un mélange de nucléosides naturels.

Les conditions CLHP sont les suivantes :
- colonne C18 analytique (250 x 4,6 mm) chauffée à 45°C,
- éluants : A : tampon phosphate de sodium, 50 mM, pH 7 ; B : MeOH/H₂O : 95/5, v/v
- gradient : 0 % de B pendant 10 min, jusqu'à 30 % de B en 10 min, jusqu'à 80 % de B en 10 min, 5 min à 80 % de B, jusqu'à 100 % de B en 2 min.

### Marquage

Le marquage des transcrits est réalisé en utilisant différentes proportions de fluorophore. Le temps de réaction est de 30 min à température ambiante. Le marquage a été effectué sur les transcrits générés à partir de cible Mtb et/ou cible HIV, obtenus par incorporation de 100 % d'un nucléotide méthylcétone. Dans un premier temps, les transcrits marqués sont analysés par électrophorèse sur gel de polyacrylamide et visualisés sous UV, avant et après coloration au bromure d'éthidium.

### Clivage

Avant d'hybrider sur la puce à ADN un clivage des transcrits marqués est réalisé à 65°C pendant 30 min en utilisant de l'imidazole et du chlorure de manganèse (MnCl₂) à une concentration de 30 mM chacun.

### Hybridation sur puce à ADN

Après clivage, les fragments obtenus sont hybridés, détectés et analysés sur puce à ADN (Affymetrix, Santa Clara, CA, USA) selon le protocole fourni par le constructeur.

Les puces dites « myco » sont conçues pour le reséquençage de la région 213-415 de la séquence M20940 « Genbank » de l'ARN 16S de *Mycobacterium tuberculosis* (Troesch A. et al, J. Clin. Microbiol., 37(1), 49-55, 1999). Celles dites « HIV PRT 440 » sont conçues pour le reséquençage des régions RT (reverse transcriptase) et protéase du virus HIV ( Kozal M.J. et al, Nature Medecine, 2(7), 753-759, 1996).

10¹⁴ copies sont hybridées sur la puce dans le cas transcrit Mtb.

5.10¹² copies sont hybridées sur la puce dans le cas du transcrit HIV.

### IV. 2. Résultats

| ***Incorporation du nucléotide uridine(C5)-C9- méthylcétone 1 (U-COCH***_{***3***}***)*** | | |
|---|---|---|
| Cible utilisée | Rapport entre nucléotide fonctionnalisé U-COCH₃ et nucléotide naturel | Transcrits produits (copies / microlitres) |
| Mtb | 0% | 5, 0^{E}+13 |
| | 30% | 5,0^{E}+13 |
| | 70% | 3,0^{E}+14 |
| | 100% | 0,8^{E}+14 |
| HIV | 0% | 0,9^{E}+12 |
| | 70% | 0,9^{E}+12 |
| | 100% | 0,9^{E}+12 |

Les résultats du tableau ci-dessus montrent que le nucléotide uridine-méthylcétone (1) est très bien incorporé par la T7 RNA polymérase. Ceci permet la production de transcrits activés capables de réagir avec un marqueur portant la fonction alcoxyamine.

### Evaluation de l'incorporation par CLHP

Dans le profil chromatographique de hydrolysat des transcrits contenant 100% de U-COCH3 (1) et obtenus à partir de cible Mtb, on retrouve bien le pic qui correspond au nucléoside U-méthylcétone (temps de rétention 26,2 minutes) ce qui signifie que le nucléotide n'est pas modifié pendant les étapes de transcription et de digestion enzymatique. On remarque aussi l'absence du pic correspondant à l'uridine naturelle (temps de rétention 14,97 minutes).

Ceci montre que le nucléotide U-méthylcétone est bien incorporé et peut être utilisé à 100% dans une réaction de transcription.

### Evaluation du marquage par gel de polyacrylamide

Les marquages ont été réalisés sur les transcrits bruts générés à partir de cible Mtb en utilisant 100% du nucléotide U-COCH₃ (1). On a fait varier la quantité en fluorophore par rapport au nombre de sites réactifs. Ces rapports sont : 2, 5, 10, 15, 20 et 50 équivalents de flurophore-ONH₂. 10 équivalents conviennent pour avoir un marquage intense et sélectif.

Sur un gel de marquage en utilisant 5, 10 et 15 eq. du fluorophore avant coloration au BET, on observe un marquage plus ou moins intense selon le nombre d'équivalents de fluorophore utilisé mais quelque soit la quantité de marqueurs utilisée, une bande est visible. On remarque aussi l'apparition de nouvelles bandes à forte dose de fluorophore. Ceci est certainement dû à une densité importance du marqueur sur la cible.

### Hybridation et analyse des résultats

Les transcrits Mtb et HIV générés à partir de cibles correspondantes, utilisant 100% d' UTP-COCH3 (1), ont subi les traitements décrits précédemment. Pendant l'étape de clivage, un agent « bloquant » (acetone ou glutaraldéhyde) a été utilisé afin d'eviter une saturation de la puce par l'excès de fluorophore-ONH₂ qui s'adsorbe sur la surface de celle-ci. L'hybridation est réalisée en utilisant la station d'hybridation Gene Chip fluidics Station (800101 Affymetrix, Santa Clara, CA), les puces et les tampons appropriées, et le protocole fourni par le constructeur.

Les paramètres pourcentage des bases appelées (Base Call), intensité moyenne des signaux, intensité médiane et le bruit de fond ont été calculés en utilisant le logiciel fourni par le constructeur (GenChip sequence analysis system, référence 900135, Affymetrix, Santa Clara, CA). Les résultats sont donnés dans les deux tableaux ci-dessous. L'intensité du signal est exprimée en RFU (unités de fluorescence du constructeur).

| *Transcrits Mtb :* | | | |
|---|---|---|---|
| Cible | Bases appelées (%) | Intensité Moyenne (Rfu) | Intensité médiane (Rfu) |
| • ***Mycobacterium Tuberculosis*** *Transcrits 100% U-COCH*_{*3*} | | | |
| 100eq Fluo-ONH₂ + 100 eq acétone | 97,7 | 3540 | 3260 |
| 20 eq Fluo-ONH2 + 20 eq glutaraldéhyde | 92,4 | 12630 | 11970 |
| Base appelée : pourcentage de bases correctement identifiées. | | | |

| *Transcrits HIV* | | | |
|---|---|---|---|
| Cible | Bases appelées (%) | Intensit é moyenne (Rfu) | Intensité Médiane (Rfu) |
| ***• HIV*** *Transcrits 70% U-COCH*_{*3*} 10 eq Fluo-ONH₂ + 10 eq glutaraldéhyde | 98,8 | 2175 | 1765 |
| *Transcrits 100% U-COCH*_{*3*} 10 eq Fluo-ONH₂ + 10 eq glutaraldéhyde | 99,0 | 2675 | 2090 |
| *Transcrits100% U-COCH*_{*3*} 5 eq Fluo-ONH₂ sans bloquant | 97,5 | 4750 | 4095 |
| | | | |
| *Transcrits 100% U-COCH*_{*3*} 5 eq Fluo-ONH₂ + 5 eq glutaraldéhyde | 98,5 | 1825 | 1550 |
| *Transcrits 100% U-COCH*_{*3*} 2 eq Fluo-ONH₂ sans bloquant | 98,3 | 2380 | 2035 |
| | | | |
| *Transcrits 100% U-COCH*_{*3*} 2 eq Fluo-ONH₂ + 2 eq glutaraldéhyde | 98,8 | 620 | 540 |

Dans les deux cas Mtb et HIV, on observe un marquage très efficace avec des intensités supérieures à 1000 Rfu et des pourcentages de reséquençage (bases appelées) voisins de 100%. Ces résultats montrent que la réaction entre l'alcoxyamine et méthylcétone, démontrée au stade monomère, est aussi spécifique et efficace entre des séquences ARN contenant des fonctions méthylcétone et le marqueur fluorescent portant la fonction alcoxyamine. En effet, avec seulement 2 équivalents de marqueur alcoxyamine par fonction méthylcétone, on obtient suffisamment de fragments de transcrits marqués et détectables.

### EXEMPLE V : INCORPORATION DE LA CYTIDINE-METHYLCETONE 3 ET MARQUAGE POST-TRANSCRIPTION

La cytidine 3 a été incorporée à 100% dans des ARN transcrits de Mtb comme décrit dans l'exemple III. Le procédé de clivage et de marquage est aussi réalisés comme indiqué précédemment.

| Cible | Bases appelées (%) | Intensité moyenne (Rfu) | Intensité médiane (Rfu) |
|---|---|---|---|
| *Transcrits 100% C-COCH*_{*3*} 20 eq Fluo-ONH₂ + 20 eq glutaraldéhyde | 94,2 | 6655 | 6200 |

Là aussi, nous observons un marquage très efficace. L'intensité du signal est supérieure à 6000 Rfu et le reséquençage est de 94%. Comme dans l'exemple précédant, ce résultat démontre que la réaction entre l'alcoxyamine et méthylcétone est spécifique et efficace entre des séquences ARN contenant des fonctions méthylcétones et le marqueur fluorescent portant la fonction alcoxyamine.

### EXEMPLE VI : AVANTAGES DE LA FONCTION METHYLCETONE PAR RAPPORT AUX FONCTIONS ALCOXYAMINES, ALDEHYDES, AMINES.

### 1. Comparaison entre la fonction alcoxyamine et méthylcétone sur le nucléotide U.

L'uridine portant une chaîne alcoxyamine en position 5 est préparée selon la méthode décrite dans l'exemple 4 de la demande de brevet WO-A-98/05766. La synthèse du marqueur fluorescéine (FLUO-CHO) aldéhyde est décrite dans l'exemple 18 de la demande WO-A-98/05766.

Il est important de noter que la fonction alcoxyamine nécessite une protection permanente par le groupement tert-butoxycarbonyl (BOC). Cette fonction est déprotégée en même temps que l'isopropylidène en 2',3' après phosphorylation en présence de 50% d'acide trifluoroacétique. Ce pourcentage en TFA est nécessaire pour obtenir un déprotection de l'alcoxyamine proche de 90%. Pour obtenir une déprotection totale, des solutions acides plus concentrées sont nécessaires. Dans ces solutions le triphosphate se dégrade très rapidement en diphosphate et monophosphate.

Dans le cas de la fonction méthylcétone, aucune déprotection n'est nécessaire et seulement 25% en TFA sont utilisés pour la déprotection totale de l'isopropylidène en 2',3' après phosphorylation. Avec ce pourcentage en TFA, aucune dégradation n'est observée.

La chaine méthylcétone est suffisamment hydrophobe même après déprotection, elle contribue ainsi à une bonne séparation du nucléotide triphosphate lors de sa purification par CLHP en phase inverse. Cette séparation est plus difficile dans le cas de la chaîne alcoxyamine.

Incorporation et post-marquage de l'UTP-alcoxyamine

Les réactions de transcription, d'hydrolyse enzymatique des transcrits, d'analyse CLHP et de post-marquage sont réalisées dans les mêmes conditions que celles décrites dans l'exemple IV.

La figure montre les rendements de transcription mesurés par la quantité d'amplicons produits (en ordonnée en copies/microlitres) en fonction des 3 nucléotides utilisés.

Dans cet exemple, l'incorporation des deux nucléotides, alcoxyamine (U-ONH₂) et méthylcétone (U-COCH₃ composé 1), est étudiée.

L'uridine méthylcétone 1 à 100% n'a aucun effet sur le rendement de transcription. Ce rendement est plus faible lors de l'incorporation du même nucléotide portant la fonction alcoxyamine.

| Post-marquage : | | | |
|---|---|---|---|
| *CIBLE MTB* | Bases appelées (%) | Intensité moyenne (Rfu) | Intensité médiane (Rfu) |
| *TRANSCRITS 100% DE U-ONH*_{*2*} *+ 10EQ FLUO-CHO* | 91,6 | 5735 | 5335 |

Le pourcentage du reséquençage en utilisant l'uridine-alcoxyamine est plus faible que celui obtenu avec son homologue portant la fonction méthylcétone.

### 2. Comparaison entre la fonction aldéhyde et méthylcétone sur le nucléotide U.

L'uridine à chaîne aldéhyde (U-CHO) est préparée selon le schéma suivant :

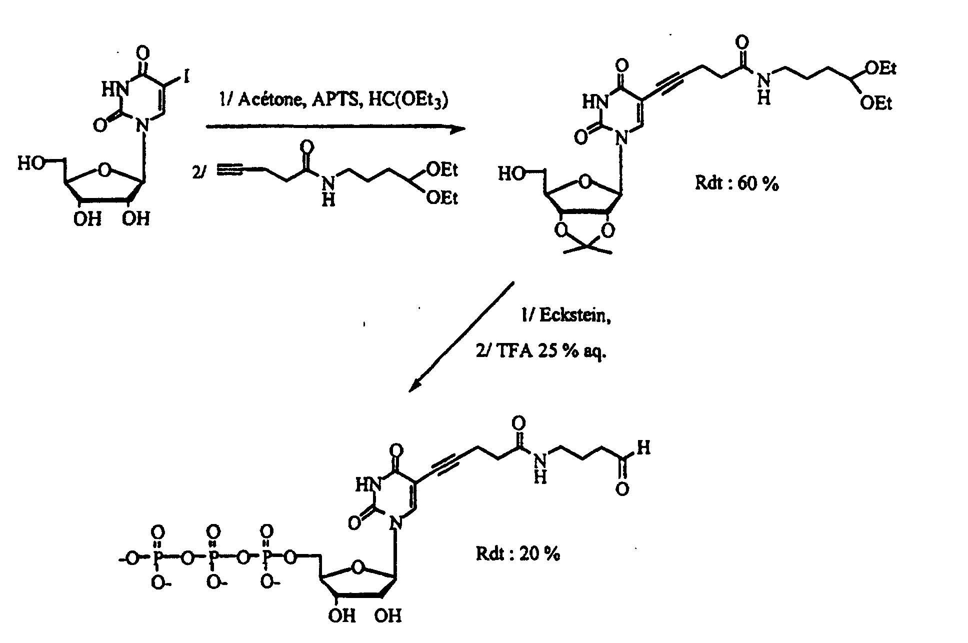

La chaîne est synthétisée par couplage peptidique entre le 4-aminobutyraldéhyde-diéthylacétal et l'acide 4-pentynoique. Le couplage de Heck conduit au nucléoside protégé. La phosphorylation suivie d'une déprotection en milieu acide conduit bien au nucléotide attendu. Les produits ont été caractérisés par RMN ¹H et RMN du ¹³C.

Là encore, la protection de la fonction aldéhydique est nécessaire lors des étapes de couplage et de phosphorylation. La déprotection de l'aldéhyde est réalisée en même temps que celle de l'isopropylidène (25% de TFA), cependant la purification et dessalage du produit déprotégé est plus difficile par rapport à celle du nucléotide méthylcétone (U-COCH₃, composé 1). Le rendement de l'étape de phosphorylation est de 20%.

L'analyse CLHP des transcrits contenant l'UTP-aldéhyde, hydrolysés par la technique enzymatique décrite dans l'exemple IV, montre que le nucléotide aldéhyde est transformé. Cette transformation est certainement due à des interactions de la fonction aldéhyde avec des composants des tampons lors de la transcription ou de d'hydrolyse enzymatique des transcrits.

La réaction du post-marquage, utilisant la fluorescéine-alcoxyamine, ne montre aucun résultat après analyse sur gel et sur puce à ADN des transcrits de mycobacterium. Ceci montre que la transformation de l'aldéhyde est survenue lors de l'étape de transcription ce qui montre l'intérêt de la fonction méthylcétone par rapport à l'aldéhyde.

### 3. Comparaison entre la fonction amine et méthylcétone sur le nucléotide U.

L'uridine portant une chaîne amine en position 5 (U-NH2) est préparée selon la méthode décrite dans l'exemple 3 de la demande de brevet WO 98/05766. La protection de la fonction amine par le groupement Boc est dans ce cas aussi nécessaire.

Les rendements de transcription obtenus avec 100% de U-NH2 sont beaucoup plus faibles que dans le cas de l'utilisation de la fonction méthylcétone et ne permettent pas un reséquençage efficace sur la puce à ADN aussi bien dans le cas de Mtb que dans le cas de HIV.

### 4. Comparaison entre la fonction amine et méthylcétone sur le nucléotide C.

La cytidine portant une chaîne amine en position 4 (C-NH₂) est préparée selon l'exemple 16 de la demande de brevet WO-A-98/05766.

Ce nucléotide a été incorporé dans des fragments de l'ARN 16S de *Mycobacterium tuberculosis* par des réactions de transcription réalisées à partir de cible PCR selon le protocole décrit dans l'exemple IV.

L'analyse CLHP des transcrits contenant la C-NH₂, hydrolysés par la technique enzymatique décrite dans l'exemple précédent, montre que le nucléotide aminé est bien incorporé et est intact.

Les rendements de transcription avec différents pourcentages de C-NH₂, aussi déterminés comme indiqué dans l'exemple IV, sont donnés dans le tableau ci-dessous.

| Rapport entre nucléotide fonctionnalisé C-NH₂ et nucléotide naturel (%) | Transcrits produits (copies / microlitres) |
|---|---|
| 0 | 3,48^{E}+13 |
| 70 | 1,94^{E}+13 |
| 100 | 1,51^{E}+12 |

Il est important de noter qu'avec 100% de C-NH₂, la quantité d'amplicons obtenus est 20 fois inférieure à celle obtenue avec une transcription contenant 100% de nucléotide naturel.

Dans la réaction de post-marquage nous avons utilisé comme réactif de marquage l'isothiocyanate de fluorescéine obtenu de chez Sigma-Aldrich (St Quentin Falavier, France).

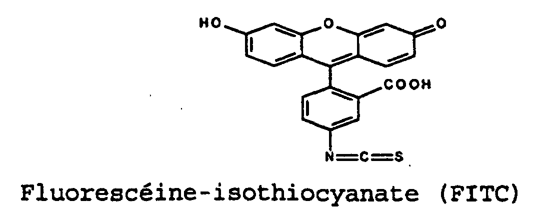

Le marquage des transcrits obtenus par incorporation de la C-NH₂ ont été marqués par la FITC, hybridés sur la puce à ADN destinée à l'identification de ces cibles Mtb et les signaux sont analysés selon le protocole décrit précédemment. Les résultats obtenus avec et sans agent bloquant sont donnés dans le tableau ci-dessus.

| *Cible* | Bases appelées (%) | Intensité moyenne (Rfu) |
|---|---|---|
| *Transcrits non purifiés* + *100eq FITC* | ND | ND |
| *Transcrits non purifiés* + *100eq FITC +1000eq de diamine bloquant* | 86 | 346 |
| ND : non déterminé, la surface de la puce est complètement saturée. | | |
| Diamine bloquant : H₂N-CH₂-CH₂-O-CH₂-CH₂-O-CH₂-CH2-NH₂. | | |

Sans addition de chaîne diamine après le marquage, la surface de la puce est complètement saturée par les 100eq de marqueur et les signaux sont inexploitables. Cette saturation est certainement due à l'interaction de la fonction isothiocyanate avec les fonction amines exocycliques des bases.

L'addition post-marquage de 1000eq de diamine, ayant la même structure que celle porté par la cytidine, semble confirmer cette hypothèse car la surface de la puce n'est plus saturée et 86% de reséquençage sont obtenus. L'intensité du signal est faible par rapport à celle obtenue avec des transcrits contenant la méthylcétone et marqués à la fluorescéine-alcoxyamine.

En plus de son effet inhibiteur lors de la transcription, le pourcentage du reséquençage utilisant le C-NH2 est plus faible que celui obtenu avec son homologue portant la fonction méthylcétone (C-COCH₃, composé 3).

## Revendications

1. Composé fonctionnalisé de formule générale (I) : 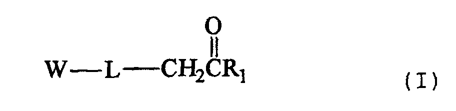 dans laquelle
W représente un analogue nucléotidique choisi parmi un nucléoside et un nucléotide, lesdits nucléoside et nucléotide pouvant porter une ou plusieurs modifications sur un de leurs éléments constitutifs que sont le sucre ribose ou désoxyribose, la base azotée et le phosphate ou son équivalent,
L représente un bras de liaison comportant un enchaînement de 8 à 30 atomes, et L est une chaîne hydrocarbonée, saturée ou insaturée, éventuellement interrompue par au moins une fonction choisie parmi les fonctions amine, amide et oxy,
R₁ représente une chaîne alkyle, linéaire ou ramifiée.

2. Composé selon la revendication 1, **caractérisé en ce que** R₁ représente une chaîne alkyle ayant au plus 6 atomes de carbone.

3. Composé selon la revendication 2, **caractérisé en ce que** R₁ représente le groupe méthyle.

4. Composé selon l'une quelconque des revendications 1 à 3, **caractérisé en ce que** W répond à la formule générale (II) 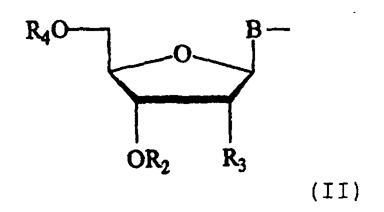 dans laquelle :
- R₂ représente H ou un groupement protecteur,
- R₃ représente H, F, OH, SH, NH₂, OCH₃ ou OR₅ où R₅ représente un groupement protecteur ou une chaîne alkyle,
- R₄ représente un radical H, un groupement protecteur ou un groupement mono, di ou triphosphate,
- B représente une base azotée,
- W étant lié à L par l'intermédiaire de B.

5. Composé selon la revendication 4, **caractérisé en ce que** la base azotée est la cytosine, l'uracile ou l'adénine.

6. Composé selon les revendications 4 et 5, **caractérisé en ce que** R₂ est un H, R₃ est un groupement OH et R₄ est un groupement triphosphate.

7. Composé selon les revendications 4 et 5, **caractérisé en ce que** R₂ est un groupement 2-cyanoéthyl-N,N-diisopropylphosphoramidite et R₃ est H ou OR₅ où R₅ est un groupement protecteur utilisé en synthèse d'oligoribonucléotide, et R₄ est un groupement 4,4'-diméthoxytrityle.

8. Polynucléotide fonctionnalisé comprenant au moins un composé fonctionnalisé selon l'une quelconque des revendications précédentes.

9. Polynucléotide fonctionnalisé selon la revendication 8, **caractérisé en ce que** ce polynucléotide est préparé par voie chimique et/ou enzymatique.

10. Polynucléotide fonctionnalisé selon la revendication 9, **caractérisé en ce que** ce polynucléotide est préparé par une réaction d'amplification enzymatique.

11. Polynucléotide fonctionnalisé marqué, issu du couplage entre un réactif de marquage portant une fonction nucléophile et la fonction alkylcétone d'un polynucléotide fonctionnalisé selon l'une quelconque des revendciations 8 à 10.

12. Polynucléotide selon la revendication 11, **caractérisé en ce que** le réactif de marquage comporte une fonction hydrazine ou alcoxyamine.

13. Polynucléotide selon la revendication 12, caratérisé en ce que le réactif de marquage est

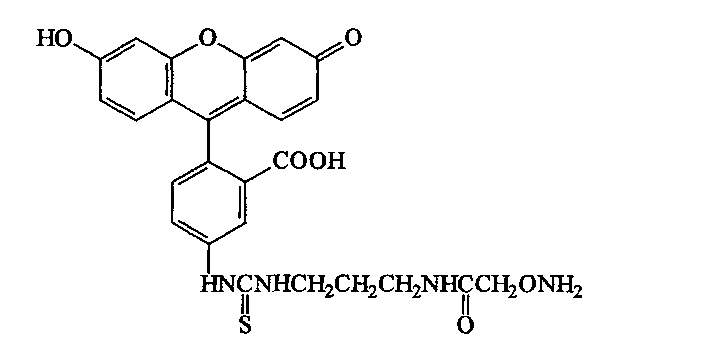

14. Procédé de détection d'un acide nucléique cible, **caractérisé en ce que**,
- l'on met en contact cet acide nucléique cible avec au moins un nucléotide fonctionnalisé de formule I telle que définie dans la revendication 1,
en présence d'au moins une enzyme pour permettre l'incorporation d'au moins un nucléotide fonctionnalisé dans l'acide nucléique cible et obtenir un acide nucléique cible fonctionnalisé,
- l'on fait réagir, sur la fonction alkylcétone de l'acide nucléique fonctionnalisé, un réactif de marquage portant une fonction nucléophile,
- et l'on détecte ledit acide nucléique marqué.

15. Procédé de détection d'un acide nucléique cible, selon la revendication 14, **caractérisé en ce que** la fonction nucléophile du réactif de marquage est une fonction alcoxyamine.

16. Procédé selon la revendication 15, **caractérisé en ce que** l'acide nucléique cible fonctionnalisé est obtenu par une réaction d'amplification enzymatique.

17. Procédé de détection d'un acide nucléique cible, **caractérisé en ce que** :
- l'on met en contact cet acide nucléique cible avec un polynucléotide fonctionnalisé comprenant au moins un composé fonctionnalisé de formule I telle que définie dans la revendication 1,
- l'on fait réagir sur la fonction alkylcétone du polynucléotide fonctionnalisé un réactif de marquage portant sur une fonction nucléophile,
- et on met en évidence la présence de l'acide nucléique cible par la détection de l'hybride marqué.

18. Procédé de détection d'un acide nucléique cible selon la revendication 17, **caractérisé en ce que** la fonction nucléophile du réactif de marquage est une fonction alcoxyamine.

19. Procédé de détection d'un acide nucléique cible, **caractérisé en ce que** :
- l'on dispose d'un polynucléotide fonctionnalisé marqué issu du couplage entre un réactif de marquage portant une fonction nucléophile et la fonction alkylcétone d'un polynucléotide fonctionnalisé comprenant au moins un composé fonctionnalisé de formule I telle que définie dans la revendication 1,
- on met en contact cet acide nucléique cible avec le polynucléotide fonctionnalisé marqué
- l'on forme un hybride entre la cible et le polynucléotide fonctionnalisé marqué,
- et l'on détecte la présence de l'acide nucléique cible par la détection de l'hybride marqué.

## Patentansprüche

1. Funktionalisierte Verbindung mit der allgemeinen Formel (I): 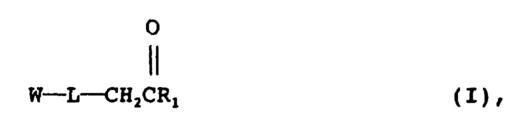 in welcher
W ein Nucleotidanalog darstellt, welches ausgewählt ist aus einem Nucleosid und einem Nucleotid, wobei das Nucleosid und das Nucleotid eine oder mehrere Modifikationen an einem ihrer Grundelemente tragen können, welche der Ribose- oder der Desoxyribosezucker, die stickstoffhaltige Base und das Phosphat oder eines seiner Äquivalente sind,
L einen Verbindungsarm mit einer Verkettung von 8 bis 30 Atomen darstellt, und L eine gesättigte oder ungesättigte Kohlenwasserstoffkette ist, die ggf. durch zumindest eine Funktion unterbrochen ist, die ausgewählt ist aus einer Amin-, Amid- und Oxid-Funktion, und
R₁ eine lineare oder verzweigte Alkylkette darstellt.

2. Verbindung nach Anspruch 1, **dadurch gekennzeichnet, dass** R₁ eine Alkylkette mit höchstens 6 Kohlenstoffatomen darstellt.

3. Verbindung nach Anspruch 2, **dadurch gekennzeichnet, dass** R₁ eine Methylgruppe darstellt.

4. Verbindung nach einem der Ansprüche 1 bis 3, **dadurch gekennzeichnet, dass** W der allgemeinen Formel (II) 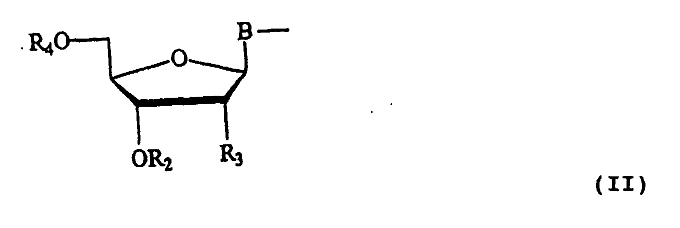 entspricht, in der:
- R₂ für H oder eine Schutzgruppe steht,
- R₃ für H, F, OH, SH, NH₂, OCH₃ oder OR₅ steht, wobei R₅ für eine Schutzgruppe oder eine Alkylkette steht,
- R₄ für einen H-Rest, eine Schutzgruppe oder eine Mono-, Di- oder Triphosphat-Gruppe steht,
- B eine stickstoffhaltige Base darstellt und
- W über B an L gebunden ist.

5. Verbindung nach Anspruch 4, **dadurch gekennzeichnet, dass** die stickstoffhaltige Base Cytosin, Uracil oder Adenin ist.

6. Verbindung nach Anspruch 4 oder 5, **dadurch gekennzeichnet, dass** R₂ ein H, R, eine OH-Gruppe und R₄ eine Triphosphat-Gruppe ist.

7. Verbindung nach Anspruch 4 oder 5, **dadurch gekennzeichnet, dass** R₂ eine 2-Cyanoethyl-N,N-diisopropylphosphoamidit-Gruppe ist, und R₃ für H oder OR₅ steht, wobei R₅ eine Schutzgruppe ist, die bei der Synthese eines Oligoribonucleotids eingesetzt wird, und dass R, eine 4,4'-Dimethoxytrityl-Gruppe ist.

8. Funktionalisiertes Polynucleotid mit zumindest einer funktionalisierten Verbindung nach einem der vorstehenden Ansprüche.

9. Funktionalisiertes Polynucleotid nach Anspruch 8, **dadurch gekennzeichnet, dass** das Polynucleotid auf chemische und/oder auf enzymatische Weise hergestellt wird.

10. Funktionalisiertes Polynucleotid nach Anspruch 9, **dadurch gekennzeichnet, dass** das Polynucleotid durch eine enzymatische Amplifikationsreaktion hergestellt wird.

11. Markiertes funktionalisiertes Polynucleotid, welches aus einer Kopplung zwischen einem Markierungsreagens, das eine nucleophile Funktion trägt, und der Alkylketonfunktion eines funktionalisierten Polynucleotids nach einem der Ansprüche 8 bis 10 entsteht.

12. Polynucleotid nach Anspruch 11, **dadurch gekennzeichnet, dass** das Markierungsreagens eine Hydrazin- oder Alkoxyamin-Funktion aufweist.

13. Polynucleotid nach Anspruch 12, **dadurch gekennzeichnet, dass** das Markierungareagens 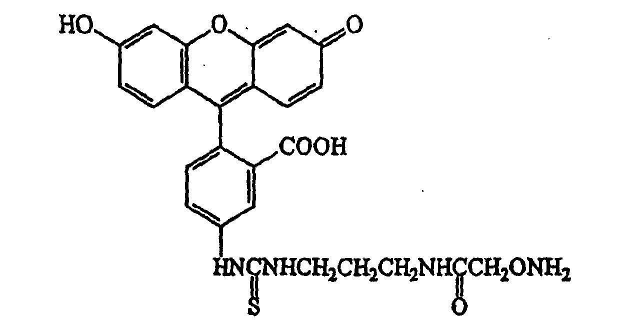 ist.

14. Verfahren zur Detektion einer Ziel-Nucleinsäure, **dadurch gekennzeichnet, dass**
- die Ziel-Nucleinsäure mit zumindest einem funktionalisierten Nucleotid der Formel (I), wie es in Ansprüche 1 definiert ist,
in Gegenwart von zumindest einem Enzym in Kontakt gebracht wird, damit zumindest ein funktionalisiertes Nucleotid in die Ziel-Nucleinsäure eingebaut und eine funktionalisierte Ziel-Nucleinsäure gewonnen werden kann,
- dass man ein Markierungsreagens, welches eine nucleophile Funktion trägt, an der Alkylketon-Funktion der funktionalisierten Nucleinsäure reagieren lässt,
- und dass die markierte Nucleinsäure detektiert wird.

15. Verfahren zur Detektion einer Ziel-Nucleinsäure nach Anspruch 14, **dadurch gekennzeichnet, dass** die nucleophile Funktion des Markierungsreagens eine Alkoxyamin-Funktion ist.

16. Verfahren nach Anspruch 15, **dadurch gekennzeichnet, dass** die funktionalisierte Ziel-Nucleinsäure durch eine enzymatische Amplifikationsreaktion gewonnen wird.

17. Verfahren zur Detektion einer Ziel-Nucleinsäure, **dadurch gekennzeichnet, dass**
- die Ziel-Nucleinsäure mit einem funktionalisierten Polynucleotid in Kontakt gebracht wird, das zumindest eine funktionalisierte Verbindung der Formel (I), wie es in Ansprüche 1 definiert ist,
- dass man ein Markierungsreagens, welches eine nucleophile Funktion trägt, an der Alkylketon-Funktion des funktionalisierten Polynucleotids reagieren lässt,
- und dass das Vorliegen der Ziel-Nucleinsäure durch die Detektion des markierten Hybrides nachgewiesen wird.

18. Verfahren zur Detektion einer Ziel-Nucleinsäure nach Anspruch 17, **dadurch gekennzeichnet, dass** die nucleophile Funktion des Markierungs-Reagens eine Alkoxyamin-Funktion ist.

19. Verfahren zur Detektion einer Ziel-Nucleinsäure, **dadurch gekennzeichnet, dass**
- ein markiertes, funktionalisiertes Polynucleotid bereitgestellt wird, das aus einer Kopplung zwischen einem Markierungsreagens, das eine nucleophile Funktion trägt und der Alkylketon-Funktion eines funktionalisierten Polynucleotids stammt, welches zumindest eine funktionalisierte Verbindung der Formel (I), wie es in Ansprüche 1 defeniert ist,
- dass diese Ziel-Nucleinsäure mit dem markierten, funktionalisierten Polynucleotid in Kontakt gebracht wird,
- dass ein Hybrid aus dem Ziel und dem markierten, funktionalisierten Polynucleotid gebildet wird,
und dass das Vorliegen der Ziel-Nucleinsäure durch die Detektion des markierten Hybrids detektiert wird.

## Claims

1. Functionalized compound of general formula (I): 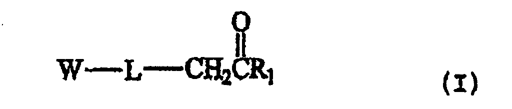 in which
W represents a nucleotide analogue chosen from a nucleoside and a nucleotide, said nucleoside and nucleotide possibly carrying one or more modifications on one of their constituent elements, namely the ribose or deoxyribose sugar, the nitrogenous base and the phosphate or its equivalent,
L represents an attachment arm comprising a series of 8 to 30 atoms, and L is a saturated or unsaturated, hydrocarbon-based chain optionally interrupted by at least one function chosen from amine, amide and oxy functions,
R₁ represents a linear or branched alkyl chain.

2. Compound according to Claim 1, **characterized in that** R₁ represents an alkyl chain having at most 6 carbon atoms.

3. Compound according to Claim 2, **characterized in that** R₁ represents the methyl group.

4. Compound according to any one of Claims 1 to 3,
**characterized in that** W corresponds to general formula (II) 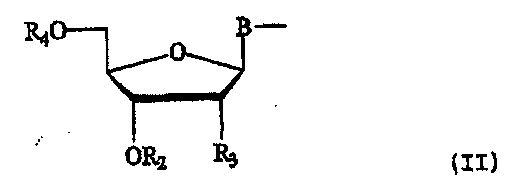 in which:
- R₂ represents H or a protective group,
- R₃ represents H, F, OH, SH, NH₂, OCH₃ or OR₅ where R₅ represents a protective group or an alkyl chain,
- R₄ represents a radical H, a protective group or a mono-, di- or triphosphate group,
- B represents a nitrogenous base,
- W being attached to L via B.

5. Compound according to Claim 4, **characterized in that** the nitrogenous base is cytosine, uracil or adenine.

6. Compound according to Claims 4 and 5, **characterized in that** R₂ is an H, R₃ is an OH group and R₄ is a triphosphate group.

7. Compound according to Claims 4 and 5, **characterized in that** R₂ is a 2-cyanoethyl-N,N-diisopropylphosphoramidite group and R₃ is H or OR₅ where R₅ is protective group used in oligoribonucleotide synthesis, and R₄ is a 4,4'-dimethoxytrityl group.

8. Functionalized polynucleotide comprising at least one functionalized compound according to any one of the

9. Functionalized polynucleotide according to Claim 8, **characterized in that** this polynucleotide is prepared via the chemical and/or enzymatic route.

10. Functionalized polynucleotide according to Claim 9, **characterized in that** this polynucleotide is prepared by an enzymatic amplification reaction.

11. Labelled functionalized polynucleotide, derived from coupling between a labelling reagent carrying a nucleophilic function and the alkyl ketone function of a functionalized polynucleotide according to any one of Claims 8 to 10.

12. Polynucleotide according to Claim 11, **characterized in that** the labelling reagent comprises a hydrazine or alkoxyamine function.

13. Polynucleotide according to Claim 12, **characterized in that** the labelling reagent is

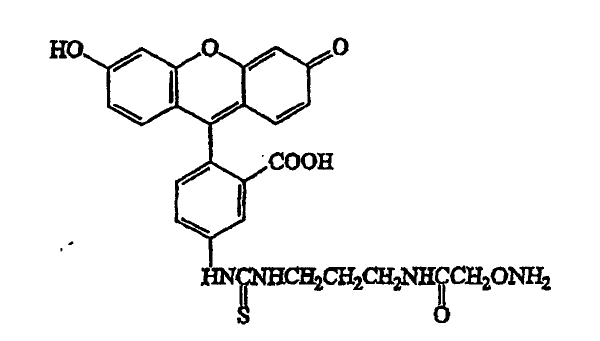

14. Method for detecting a target nucleic acid, **characterized in that**
- this target nucleic acid is brought into contact with at least one functionalized nucleotide of formula I, as defined in claim 1, in the presence of at least one enzyme so as to allow the incorporation of at least one functionalized nucleotide into the target nucleic acid and to obtain a functionalized target nucleic acid,
- a labelling reagent carrying a nucleophilic function is reacted with the alkyl ketone function of the functionalized nucleic acid,
- and said labelled nucleic acid is detected.

15. Method for detecting a target nucleic acid according to Claim 14, **characterized in that** the nucleophilic function of the labelling reagent is an alkoxyamine function.

16. Method according to Claim 15, **characterized in that** the functionalized target nucleic acid is obtained by an enzymatic amplification reaction.

17. Method for detecting a target nucleic acid, **characterized in that**:
- this target nucleic acid is brought into contact with a functionalized polynucleotide comprising at least one functionalized compound of formula I, as defined in claim 1,
- a labelling reagent carrying a nucleophilic function is reacted with the alkyl ketone function of the functionalized polynucleotide,
- and the presence of the target nucleic acid is demonstrated by detection of the labelled hybrid.

18. Method for detecting a target nucleic acid according to Claim 17, **characterized in that** the nucleophilic function of the labelling reagent is an alkoxyamine function.

19. Method for detecting a target nucleic acid, **characterized in that**:
- a labelled functionalized polynucleotide is available, derived from coupling between a labelling reagent carrying a nucleophilic function and the alkyl ketone function of a functionalized polynucleotide comprising at least one functionalized compound of formula I, as defined in claim 1,
- this target nucleic acid is brought into contact with the labelled functionalized polynucleotide,
- a hybrid is formed between the target and the labelled functionalized polynucleotide,
- and the presence of the target nucleic acid is detected by detection of the labelled hybrid.
